# FASCICULE DE BREVET EUROPEEN

(11) **EP 2 623 025 B1**
(45) Date de publication et mention de la délivrance du brevet: **21.06.2017**
(21) Numéro de dépôt: 13153073.5
(22) Date de dépôt: 29.01.2013
(51) Int. Cl.: A61N 1/36, A61B 5/04, A61B 5/00

(54) **Dispositif implantable du type interface neuronale et procédé associé**
Implantierbare Vorrichtung als neuronale Schnittstelle und zugehöriges Verfahren
Implantable device of the type neural interface and assocaited method.

(30) Priorité: 02.02.2012 FR 1250999
(43) Date de publication de la demande: 07.08.2013
(73) Titulaire: Commissariat à l'Énergie Atomique et aux Énergies Alternatives, 75015 Paris (FR)
(72) Inventeur: Sauter-Starace, Fabien, 38180 Seyssins (FR); Charvet, Guillaume, 38360 Sassenage (FR); Benabid, Alim-Louis, 38240 Meylan (FR)
(74) Mandataire: Ilgart, Jean-Christophe

(56) Documents cités:
- WO-A1-02/36003
- WO-A1-2011/067297
- WO-A2-2006/041738
- WO-A2-2011/123150

## Description

### DOMAINE TECHNIQUE

La présente invention se rapporte au domaine des dispositifs implantables au contact du cerveau humain ou animal pour le traitement de signaux neuronaux. Elle concerne en particulier les implants cérébraux connus sous l'appellation d' « interface neuronale » permettant une communication entre le cerveau et un dispositif électronique, notamment la communication de signaux neuronaux enregistrés par des électrodes placées au niveau du cerveau.

L'invention concerne ainsi un dispositif implantable du type interface neuronale ainsi qu'un procédé de fabrication associé.

### ÉTAT DE LA TECHNIQUE ANTÉRIEURE

De nombreux systèmes implantables dans le cerveau humain ou animal pour la lecture, l'enregistrement et/ou le contrôle de signaux neuronaux ont été conçus depuis ces dernières années afin notamment de diagnostiquer, de prévenir, d'assister, d'améliorer ou de réparer des fonctions humaines ou animales de cognition ou des actions défaillantes.

De tels systèmes ont par exemple trouvé des applications dans le traitement de crises d'épilepsie, dans l'atténuation des symptômes de la maladie de Parkinson, dans la lutte contre la dépression et dans le traitement d'autres problèmes médicaux.

Ces systèmes peuvent notamment permettre le pilotage d'effecteurs pour des applications dites de type « Interface Neuronale Directe » (IND) ou « Brain-Computer Interface » (BCI) en anglais (interface cerveau-machine). De façon générale, les applications de type IND ou BCI peuvent permettre le pilotage d'actionneurs au moyen des signaux neuronaux collectés par des électrodes implantées dans ou sur le cerveau. On parle également de pilotage par la pensée.

Les divers implants cérébraux connus de l'art antérieur pour le traitement de signaux neuronaux peuvent être classifiés en fonction de la zone d'implantation.

La technologie de l'électrocorticographie (EcoG) caractérise les dispositifs permettant l'enregistrement graphique de l'activité cérébrale grâce à des électrodes, réparties selon une matrice, placées au contact direct du cortex sous la dure-mère. Les signaux corticaux mesurés sont traités par des moyens électroniques. Cette technique est invasive car elle requiert d'effectuer une craniotomie sur l'individu à examiner, c'est-à-dire une incision chirurgicale au niveau du crâne.

L'électro-encéphalographie (EEG) constitue une autre méthode d'exploration cérébrale qui mesure l'activité électrique du cerveau par des électrodes placées sur le cuir chevelu. Cette technique est non-invasive.

Enfin, on connaît également deux autres technologies de mesure des signaux neuronaux, et en particulier du potentiel neuronal, connues sous les appellations de « Single Unit Action Potential » (SUAP) et « Local Field Potential » (LFP) en anglais. Elles reposent sur le principe de pénétration d'électrodes en pointes dans le cerveau, en particulier dans le cortex pour y effectuer la mesure.

Les moyens électroniques permettant de recevoir les signaux neuronaux captés par les électrodes peuvent être situés à l'extérieur du cerveau et reliés aux électrodes par des liaisons filaires. Toutefois, pour optimiser le rapport signal sur bruit, il s'est avéré intéressant de vouloir les placer au plus près de la zone d'enregistrement des signaux et ainsi de les implanter également au niveau du cerveau.

L'article intitulé « BioBolt : A Minimally-Invasive Neural Interface for Wireless Epidural Recording by Intra-Skin Communication », Sun-II Chang et al, 2011 Symposium on VLSI Circuits Digest of Technical Papers, pages 146-147, et la demande internationale WO 2011/123150 décrivent une interface neuronale qui comporte une tête élargie placée au sommet d'un corps fileté. La tête est destinée à venir s'appliquer contre le crâne alors que le corps fileté est prévu pour être inséré dans un alésage de même diamètre formé dans l'os du crâne. Une électrode est disposée à la base du corps fileté, et couplée électriquement à un circuit électrique situé dans la tête élargie. Le circuit électrique peut permettre une éventuelle pré-amplification et/ou une numérisation des signaux électriques collectés par l'électrode, et leur transmission vers des moyens électroniques ou informatiques distants, par une liaison filaire ou hertzienne, pour leur traitement.

Par ailleurs, la demande internationale WO 2011/067297 A1 décrit un système implantable dans le cerveau pour la stimulation et l'enregistrement de signaux neuronaux. Dans un premier mode de réalisation, le système est placé sur le crâne et des électrodes sont au contact du cerveau dans une cavité du crâne alors que, dans un deuxième mode de réalisation, les électrodes sont disposées sous le système, lequel est placé dans la cavité formée sur le crâne.

Les dispositifs d'implants cérébraux décrits ci-dessus et qui relèvent de techniques d'implantation invasives sont des dispositifs placés au contact direct de la peau, celle-ci recouvrant le plus souvent la partie supérieure des dispositifs. Ce contact direct avec la peau peut engendrer des risques de lésions et/ou d'infection de la peau non négligeables, et ce plus particulièrement au niveau de la jonction entre le crâne et la partie supérieure du dispositif lorsque ce dernier est logé dans une cavité du crâne formée par craniotomie. De plus, l'intégration de ces dispositifs implantables sur le cerveau d'un individu peut entraîner une gêne importante pour l'individu au niveau de la zone d'implantation liée à son confort d'utilisation. Elle peut également s'avérer disgracieuse et révéler un aspect extérieur du crâne non homogène.

### EXPOSÉ DE L'INVENTION

Il existe un besoin pour concevoir un dispositif implantable du type interface neuronale pour le traitement de signaux neuronaux permettant de minimiser les risques médicaux liés à son utilisation, notamment les risques de lésions et/ou d'infection du corps d'un individu, et notamment de la peau recouvrant le crâne de l'individu. Il existe également un besoin pour permettre une intégration homogène d'un tel dispositif au niveau du cerveau afin d'obtenir un confort esthétique et physique pour l'individu.

L'invention vise à répondre à tout ou partie de ces besoins.

L'invention a ainsi pour objet, selon l'un de ses aspects, un dispositif implantable du type interface neuronale pour le traitement de signaux, notamment de signaux neuronaux, comportant :
- un boîtier destiné à être logé au moins partiellement dans une cavité formée sur le crâne d'un être humain ou animal,
- des moyens électroniques de traitement de signaux situés dans le boîtier, et
- un système d'électrodes couplé électriquement aux moyens électroniques,
caractérisé en ce que le boîtier comporte une face supérieure, destinée à venir au contact de la peau, dont la surface est sensiblement convexe.

Par « sensiblement convexe », on entend que la surface de la face supérieure est convexe, ou tout au moins apparaît comme convexe à l'oeil nu.

La convexité de la surface de la face supérieure du boîtier peut être déterminée de façon à reproduire au moins partiellement la courbure du crâne au niveau de ladite cavité et à assurer une sensible continuité de surface entre la face supérieure du boîtier et les portions de surface du crâne adjacentes. La courbure de la face supérieure du boîtier peut par exemple être équivalente à la courbure réelle du crâne de l'être humain ou animal, une telle courbure variant en fonction du crâne et de la zone d'implantation. Le rayon de courbure peut ainsi varier entre 50 et 200 mm.

Grâce à l'invention, le dispositif peut être intégré de façon homogène au niveau du crâne de l'individu et présenter un nombre nul ou sensiblement nul d'aspérités sur sa face supérieure au contact de la peau de sorte à ne pas entraîner de risques de lésions et/ou d'infection de la peau. Le dispositif peut également permettre une incorporation dans la continuité du crâne, en réduisant fortement la cassure habituelle qui existe à la jonction entre le crâne et la face supérieure du dispositif, de sorte que le dispositif soit le plus discret possible et tende à être visuellement indétectable.

Le dispositif selon l'invention peut en outre comporter l'une ou plusieurs des caractéristiques suivantes prises isolément ou suivant toutes combinaisons techniques possibles.

Le boîtier peut être formé par l'assemblage d'une partie supérieure et d'une partie inférieure, notamment par soudage.

La partie supérieure peut comporter un rebord s'étendant depuis la face supérieure du boîtier et définissant la surface latérale de la partie supérieure, le rebord étant destiné à être inséré dans la partie inférieure du boîtier, en prenant appui contre la face latérale de la partie inférieure.

Le rebord peut s'étendre sur tout le pourtour de la partie supérieure.

La partie supérieure du boîtier peut comporter des pattes de fixation, faisant notamment saillies sur le rebord, destinées à prendre appui sur le crâne de l'être humain ou animal.

La partie supérieure peut présenter une forme sensiblement circulaire ou polygonale, notamment une forme carrée.

Des méplats peuvent être formés au niveau d'angles, notamment de chaque angle, de la partie supérieure, les pattes de fixation pouvant notamment faire saillies à partir de ces méplats.

La partie inférieure peut présenter une face inférieure et une face latérale qui définissent entre elles un logement en forme de cuvette pour recevoir les moyens électroniques.

La face latérale de la partie inférieure peut être ajourée, comportant notamment des ouvertures pour permettre le passage de contacts électriques pour coupler électriquement les moyens électroniques avec le système d'électrodes.

La partie inférieure peut présenter une forme sensiblement circulaire ou polygonale, notamment une forme carrée.

Le boîtier, notamment la partie supérieure et/ou la partie inférieure, peut être surmoulé par un matériau de surmoulage, notamment une résine de silicone biocompatible.

Le boîtier peut présenter une plus grande dimension transversale comprise entre 1 et 5 cm.

L'épaisseur du boîtier peut être comprise entre 0,3 et 1 cm, notamment entre 0,5 et 1 cm.

Le boîtier peut être réalisé dans un matériau rigide et biocompatible, étant par exemple réalisé en titane ou en céramique.

Le boîtier peut être hermétique, afin notamment d'éviter toute intrusion de liquide à l'intérieur du boîtier.

Le système d'électrodes peut être fixé sous le boîtier, au niveau de la face inférieure de la partie inférieure du boîtier.

Le système d'électrodes peut être inscrit dans le périmètre externe du boîtier, notamment dans le périmètre externe de la partie inférieure du boîtier.

Le système d'électrodes peut comporter des électrodes agencées selon une matrice prédéterminée, notamment en un réseau régulier ou non.

Le système d'électrodes peut comporter des électrodes d'enregistrement de signaux, notamment de signaux neuronaux, et/ou des électrodes de stimulation. Les électrodes de stimulation peuvent par exemple permettre l'émission de signaux électriques dans le cerveau, par exemple pour traiter différents maux tels que la maladie de Parkinson, la dépression, la migraine et/ou l'obésité.

Les moyens électroniques peuvent être des moyens électroniques de traitement de signaux neuronaux mesurés par des électrodes d'enregistrement et/ou des moyens électroniques de production de signaux d'électrostimulation par des électrodes de stimulation.

Le dispositif peut en outre comporter une enveloppe de transmission de signaux, s'étendant depuis la périphérie du boîtier, notamment depuis la périphérie de la partie inférieure du boîtier.

Par « transmission de signaux », on entend la transmission de signaux neuronaux mesurés et/ou de signaux d'électrostimulation, et/ou la transmission de signaux à destination des moyens électroniques, par exemple des signaux de téléalimentation et/ou des signaux de commande pilotant ces moyens.

L'enveloppe peut ainsi comporter au moins une antenne de transmission, pour l'échange d'informations en émission et/ou réception, et/ou au moins une antenne de téléalimentation. En particulier, l'enveloppe peut comporter une antenne de transmission et une antenne de téléalimentation, les deux antennes étant coplanaires et disposées de sorte que l'une est inscrite dans l'autre.

L'enveloppe peut être réalisée dans un matériau souple et biocompatible, notamment à partir d'un élastomère de type silicone.

L'enveloppe peut présenter une épaisseur suffisamment mince pour pouvoir être glissée entre le crâne et la peau du cuir chevelu.

L'épaisseur de l'enveloppe peut notamment diminuer en éloignement du boîtier. Autrement dit, l'épaisseur de l'enveloppe peut diminuer avec la distance par rapport à la périphérie du boîtier. L'épaisseur de l'enveloppe peut par exemple être comprise entre 1 et 3 mm, étant par exemple inférieure à 1,5 mm, mieux 1 mm, mieux encore 0,7 mm. L'épaisseur de l'enveloppe peut être constante sur une distance prédéterminée à partir du boîtier, puis diminuer progressivement jusqu'à l'extrémité de l'enveloppe.

L'enveloppe peut présenter une dureté Shore A inférieure ou égale à 50.

L'enveloppe peut présenter une surface supérieure sensiblement convexe (ou courbe) de façon à reproduire au moins partiellement la courbure du crâne et/ou celle du cuir chevelu à l'endroit de son implantation, et à assurer une sensible continuité de surface entre la face supérieure du boîtier et la surface supérieure de l'enveloppe.

En particulier, la surface supérieure de l'enveloppe peut être reliée à la face supérieure du boîtier de façon continue de sorte à présenter un arrondi épousant la forme du cuir chevelu et/ou celle du crâne à l'interface entre le boîtier et l'enveloppe.

La surface supérieure de l'enveloppe et la face supérieure du boîtier peuvent être tangentes l'une par rapport à l'autre au niveau de leur interface.

L'invention a encore pour objet, selon un autre de ses aspects, un procédé d'implantation d'un dispositif tel que défini précédemment dans une cavité formée sur le crâne d'un être humain ou animal, dans lequel on place le dispositif dans la cavité de sorte que la face supérieure du boîtier et les portions de surface du crâne adjacentes au boîtier forment sensiblement une continuité de surface.

Par « continuité de surface », on entend que la jonction entre la face supérieure du dispositif, notamment celle du boîtier, et les portions de surface du crâne adjacentes ne présente sensiblement pas de surépaisseur. En particulier, la jonction entre la face supérieure du dispositif et le crâne peut présenter un aspect lisse. Ainsi, d'une façon générale, au niveau de ladite jonction, le plan tangent à la face supérieure du dispositif est parallèle, voire sensiblement confondu, au plan tangent au crâne dans lequel la face supérieure est insérée.

L'invention a encore pour objet, selon un autre de ses aspects, un procédé de fabrication d'un dispositif, et notamment d'un boîtier, tel que défini précédemment dans lequel on détermine la convexité de la face supérieure du boîtier du dispositif à partir de mesures de convexité préalablement effectuées sur le crâne d'un être humain ou animal.

L'invention a encore pour objet, selon un autre de ses aspects, un procédé de fabrication d'un dispositif implantable du type interface neuronale pour le traitement de signaux, comportant :
- un boîtier destiné à être logé au moins partiellement dans une cavité formée sur le crâne d'un être humain ou animal,
- des moyens électroniques de traitement de signaux situés dans le boîtier, et
- un système d'électrodes couplé électriquement aux moyens électroniques,
le boîtier comportant une face supérieure, destinée à venir au contact de la peau, dont la surface est sensiblement convexe,
dans lequel on détermine la convexité de la face supérieure du boîtier du dispositif à partir de mesures de convexité préalablement effectuées sur le crâne d'un être humain ou animal.

Les mesures de convexité peuvent être effectuées à l'endroit d'implantation du dispositif, notamment au niveau du crâne où sera formée la cavité pour l'implantation, par exemple par craniotomie.

L'invention a encore pour objet, selon un autre de ses aspects, un dispositif implantable du type interface neuronale pour le traitement de signaux, notamment de signaux neuronaux, comportant :
- un boîtier destiné à être logé au moins partiellement dans une cavité formée sur le crâne d'un être humain ou animal,
- des moyens électroniques de traitement de signaux situés dans le boîtier, et
- un système d'électrodes couplé électriquement aux moyens électroniques,
caractérisé en ce qu'il comporte une enveloppe de transmission de signaux s'étendant depuis la périphérie du boîtier.

Les électrodes peuvent par exemple être des électrodes d'enregistrement de signaux neuronaux ou de stimulation.

L'invention a encore pour objet, selon un autre de ses aspects, une enveloppe de transmission pour un dispositif implantable du type interface neuronale, comportant au moins une antenne de transmission et/ou au moins une antenne de téléalimentation.

L'invention a encore pour objet, selon un autre de ses aspects, un dispositif implantable du type interface neuronale pour le traitement de signaux, notamment de signaux neuronaux, comportant :
- un boîtier destiné à être logé au moins partiellement dans une cavité formée sur le crâne d'un être humain ou animal,
- des moyens électroniques de traitement de signaux situés dans le boîtier, et
- un système d'électrodes couplé électriquement aux moyens électroniques,
caractérisé en ce que le système d'électrodes est inscrit dans le périmètre externe du boîtier, le boîtier comportant notamment une face supérieure, destinée à venir au contact de la peau, dont la surface est sensiblement convexe.

Le dispositif, et notamment l'enveloppe du dispositif, peut comporter une ou plusieurs des caractéristiques mentionnées précédemment.

### BRÈVE DESCRIPTION DES DESSINS

L'invention pourra être mieux comprise à la lecture de la description détaillée qui va suivre, d'un exemple de mise en oeuvre non limitatif de celle-ci, ainsi qu'à l'examen du dessin annexé, sur lequel :
- la figure 1 est une représentation, en perspective et en vue éclatée, d'un exemple de dispositif implantable selon l'invention,
- la figure 2 représente, en perspective, le dispositif de la figure 1 dans une configuration assemblée,
- la figure 3 représente, en perspective, la partie supérieure du boîtier du dispositif des figures 1 et 2,
- la figure 4 est une vue en coupe et en perspective du dispositif des figures 1 et 2,
- la figure 5 représente, en perspective, la partie inférieure du boîtier du dispositif des figures 1 et 2,
- la figure 6 représente, en perspective et en vue du dessous, le dispositif des figures 1 et 2,
- la figure 7 représente, en vue de face, le système d'électrodes du dispositif des figures 1 et 2,
- les figures 8a et 8b illustrent deux étapes de la fabrication de l'enveloppe du dispositif de la figure 1, et
- les figures 9 et 10 illustrent, respectivement en vue en perspective et en coupe, l'implantation de deux dispositifs selon l'invention sur le crâne d'un être humain.

Dans l'ensemble de ces figures, des références identiques peuvent désigner des éléments identiques ou analogues.

### EXPOSÉ DÉTAILLÉ D'UN MODE DE RÉALISATION PARTICULIER

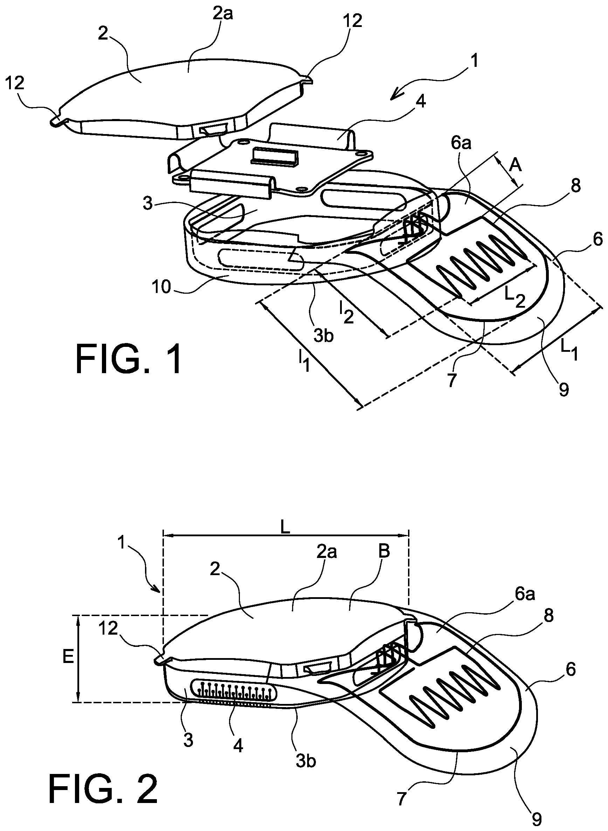

On a représenté sur les figures 1 et 2 un exemple de dispositif implantable 1 conforme à l'invention.

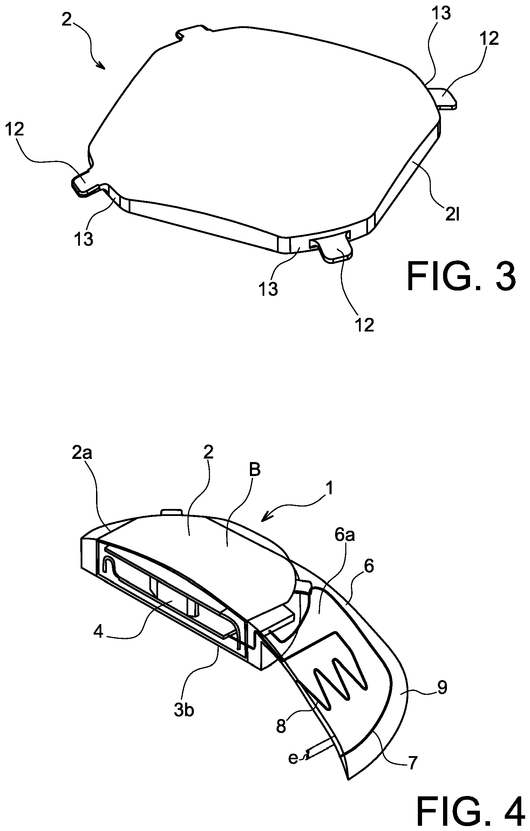

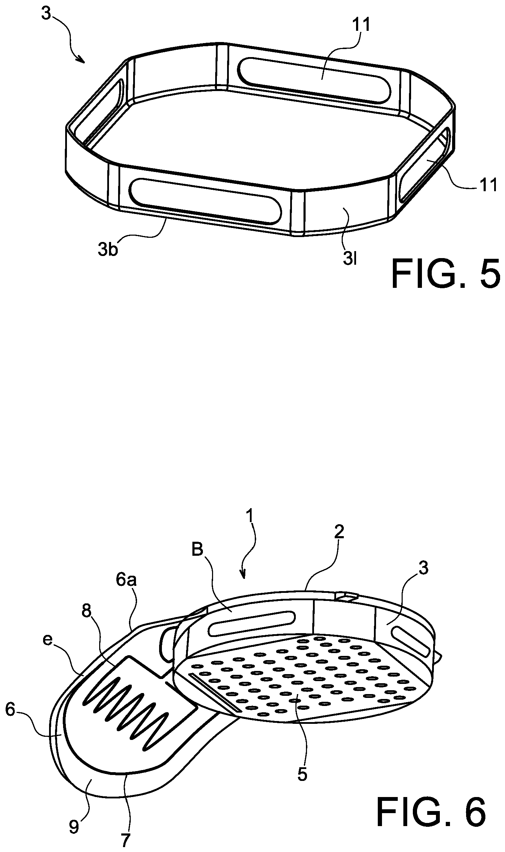

Le dispositif 1 comporte un boîtier B à l'intérieur duquel sont logés des moyens électroniques 4. Le boîtier B est formé par l'assemblage d'une partie supérieure 2 et d'une partie inférieure 3, respectivement visibles de manière isolée sur les figures 3 et 5.

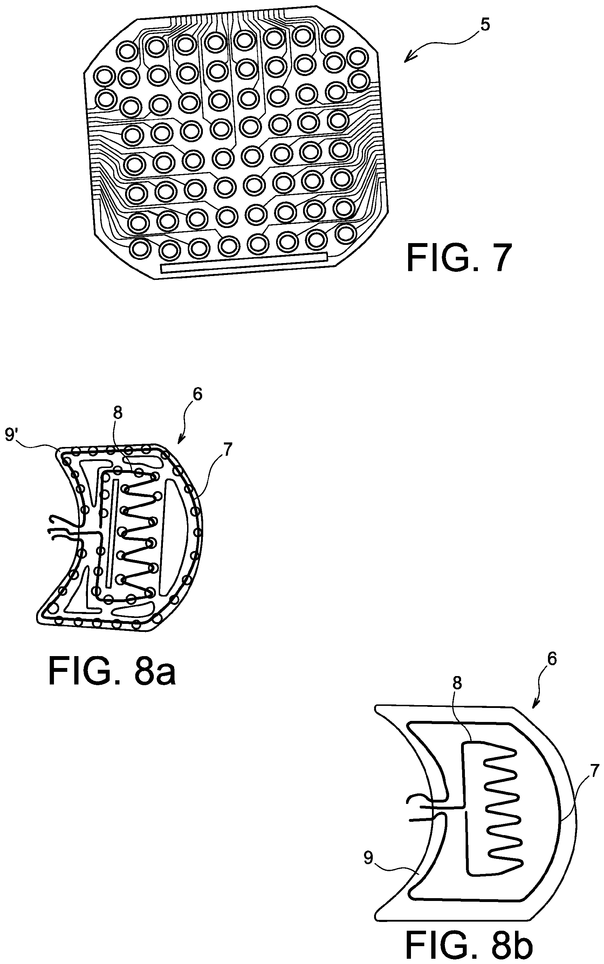

En outre, le dispositif 1 comporte un système d'électrodes 5, comme on peut le voir sur la figure 6 et de façon isolée sur la figure 7.

Le dispositif 1 comporte encore une enveloppe 6 de transmission, qui sera détaillée par la suite.

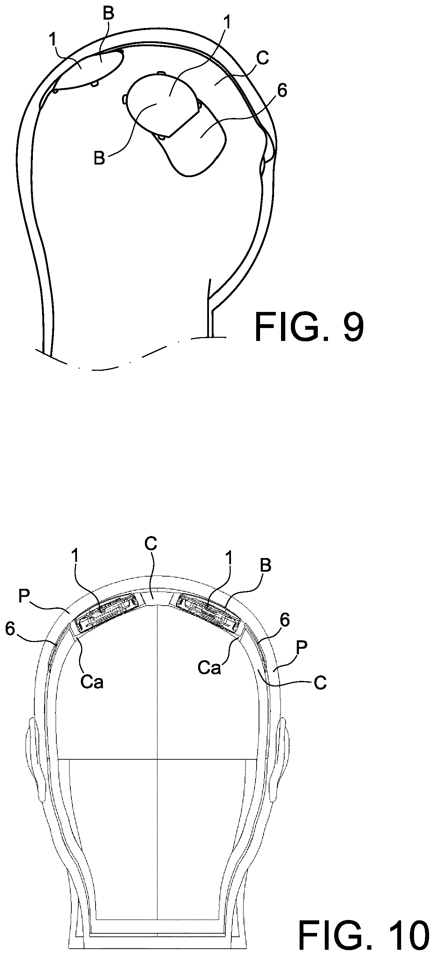

Le dispositif implantable 1, et notamment le boîtier B du dispositif 1, est destiné à être implanté au contact du cerveau d'un individu afin notamment d'enregistrer et de traiter des signaux neuronaux du cerveau. Les figures 9 et 10 illustrent, de façon schématique, l'implantation de deux dispositifs 1 conforme à l'invention respectivement dans deux cavités Ca formées sur le crâne C d'un être humain, par exemple à la suite d'une opération de craniotomie. Le boîtier B d'un dispositif 1 est logé dans une cavité Ca alors que l'enveloppe 6 de ce dispositif 1 est insérée entre le crâne C et la peau P du cuir chevelu.

Conformément à l'invention, le boîtier B comporte une face supérieure 2a, destinée à venir au contact de la peau P, dont la surface est sensiblement convexe. La convexité de la surface de la face supérieure 2a peut permettre de conférer à la partie supérieure 2 du boîtier B destinée à venir au contact de la peau P une forme arrondie de façon à reproduire au moins partiellement, voir totalement, la courbure du crâne C au niveau de la cavité Ca formée dans celui-ci et, par ailleurs, d'assurer une sensible continuité de surface entre la face supérieure 2a du boîtier B et les portions de surface du crâne C adjacentes au dispositif 1. La convexité de la face supérieure 2a peut donc permettre un lissage de l'interface entre le dispositif 1 et les portions de surface du crâne C qui lui sont adjacentes.

Avantageusement, la face supérieure 2a du boîtier B peut permettre d'épouser la forme du crâne C à l'endroit de la cavité Ca formée dans celui-ci de façon à réduire, voire à rendre inexistantes, les aspérités sur la surface de la face supérieure 2a du boîtier B, et en particulier au niveau de la jonction entre la face supérieure 2a du boîtier B et les portions de surface du crâne C adjacentes au dispositif 1. De la sorte, le risque de liaisons et/ou d'infection de la peau, notamment au niveau de cette jonction, est fortement réduit. Le dispositif 1 peut donc être implanté au contact du cerveau pour une longue durée tout en limitant les risques pour l'individu. La convexité de la face supérieure 2a du boîtier B peut en effet permettre au dispositif 1, et notamment à la face supérieure 2a de la partie supérieure 2 du boîtier B, de venir à affleurement de l'os du crâne C situé à la périphérie de la cavité Ca.

La face supérieure 2a du boîtier B peut présenter une surface convexe ou multiconvexe, en particulier biconvexe, par exemple ayant une convexité selon deux directions orthogonales. La convexité de la surface de la face supérieure 2a du boîtier B peut par exemple être déterminée par rapport à la courbure du crâne C, notamment au niveau de la cavité Ca avant sa formation, par exemple par craniotomie. En particulier, la face supérieure 2a du boîtier B peut présenter sensiblement la même courbure que celle du crâne C dans la région où la cavité Ca est formée.

La partie supérieure 2 du boîtier B comporte une face supérieure 2a convexe telle que décrite précédemment et également, comme on peut le voir sur la figure 3, un rebord 2l s'étendant depuis la face supérieure 2a et définissant la surface latérale de la partie supérieure 2. Le rebord 2l s'étend en particulier sur tout le pourtour de la partie supérieure 2 dans cet exemple mais, en variante, le rebord 2l peut ne s'étendre qu'en partie sur le pourtour de la partie supérieure 2 du boîtier B. Le rebord 2l est destiné à être inséré dans la partie inférieure 3 du boîtier B, en prenant appui, notamment du côté intérieur, contre la face latérale 3l de la partie inférieure 3.

La partie supérieure 2 du boîtier B comporte en outre des pattes de fixation 12, comme on peut le voir sur la figure 3, faisant saillies sur le rebord 21. Les pattes de fixation 12 sont par exemple situées au niveau de coins de la partie supérieure 2. Les pattes de fixation 12 sont notamment destinées à prendre appui sur le crâne C de l'individu, au niveau des portions de crâne situées à la périphérie de la cavité Ca. De la sorte, les pattes de fixation 12 peuvent permettre le maintien du dispositif 1 sur le crâne C en empêchant que celui-ci ne s'enfonce dans le crâne C au niveau de la cavité Ca. Les pattes de fixation 12 peuvent être maintenues sur le crâne C par tout type de fixation, notamment par des vis intracrâniennes. L'épaisseur des pattes de fixation 12 peut être suffisamment faible pour ne pas engendrer de surépaisseur locale à l'interface entre le dispositif 1 et les portions de crâne C adjacentes au dispositif 1 susceptibles d'entraîner des risques de lésions et/ou d'infection de la peau P.

La partie inférieure 3 présente une face inférieure 3b et une face latérale 3l qui définissent entre eux un logement en forme de cuvette pour recevoir les moyens électroniques 4. La face supérieure 2a de la partie supérieure 2 est destinée à être appliquée contre la peau P du cuir chevelu alors que la face inférieure 3b de la partie inférieure 3 est dirigée vers le cortex du cerveau de l'individu. La partie inférieure 3 peut recevoir le système d'électrodes 5, comme on peut le voir sur la figure 6. La face latérale 3l de la partie inférieure 3 est ajourée. Elle comporte en particulier des ouvertures 11, par exemple de forme oblongue, par exemple au nombre de quatre dans cet exemple. Les ouvertures 11 peuvent permettre le passage de contacts électriques pour coupler électriquement les moyens électroniques 4 avec le système d'électrodes 5.

La partie supérieure 2 forme un couvercle destiné à venir fermer le logement formé dans la partie inférieure 3 du dispositif 1 par la face latérale 3l et la face inférieure 3b de la partie inférieure 3. En particulier, la partie supérieure 2 peut être soudée sur la partie inférieure 3 du boîtier B.

La partie supérieure 2 et/ou la partie inférieure 3 peuvent présenter une forme sensiblement circulaire, notamment une forme carré. Dans ce cas, les pattes de fixation 12 de la partie supérieure 2 peuvent être situées aux quatre coins de la partie supérieure 2. Des méplats 13 peuvent être formés au niveau de chaque angle de la partie supérieure 2, notamment au niveau de chaque angle du rebord 31, et les pattes de fixation 12 peuvent faire saillies à partir de ces méplats 13.

La face latérale 3l de la partie inférieure 3 peut également présenter une forme sensiblement circulaire, voire une forme carré. Dans ce cas, les ouvertures 11 peuvent être formées sur chacun des quatre côtés définis par la face latérale 31.

Le boîtier B, notamment la partie supérieure 2 et/ou la partie inférieure 3, peut être surmoulé par un matériau de surmoulage 10, notamment une résine de silicone biocompatible. Sur la figure 1, la partie inférieure 3 est surmoulée par un tel matériau de surmoulage 10. Le matériau de surmoulage 10 peut permettre de modifier le périmètre externe du boîtier B, et notamment de la partie supérieure 2 et/ou de la partie inférieure 3, par exemple en lui conférant une forme sensiblement cylindrique.

La plus grande dimension transversale de la cavité Ca formée dans le crâne C de l'individu peut être comprise entre 1 et 5 cm. De ce fait, le boîtier B du dispositif implantable 1 peut présenter une plus grande dimension transversale L comprise entre 1 et 5 cm, étant par exemple d'environ 47 mm dans cet exemple. En outre, l'épaisseur E du boîtier B peut par exemple être comprise entre 0,3 et 1 cm, notamment entre 0,5 et 1 cm, par exemple égale à environ 0,7 cm.

Le boîtier B est de préférence réalisé dans un matériau rigide et biocompatible, étant par exemple réalisé en titane ou en céramique. En outre, le boîtier B est de préférence hermétique, afin notamment d'éviter toute intrusion de liquide, voire de gaz, à l'intérieur du boîtier B.

Les moyens électroniques 4 peuvent ainsi être logés de façon hermétique à l'intérieur du boîtier B. Les moyens électroniques 4 peuvent être isolés électriquement du boîtier B.

Les moyens électroniques 4 sont couplés électriquement au système d'électrodes 5.

Les moyens électroniques 4 peuvent par exemple comporter un préamplificateur, un convertisseur analogique numérique et/ou des moyens pour la transmission de données mesurées relatives aux signaux neuronaux.

Les moyens électroniques 4 peuvent comporter un système d'alimentation et/ou un circuit apte à générer un signal d'alimentation à partir d'un signal détecté par une antenne de téléalimentation.

La figure 6 représente le positionnement du système d'électrodes 5 sous le dispositif implantable 1, au niveau de la face inférieure 3b de la partie inférieure 3 du boîtier B.

La figure 7 représente, en vue de face, le système d'électrodes 5.

Le système d'électrodes 5 est ainsi de préférence disposé à l'extérieur du boîtier B. En particulier, la face inférieure 3b du boîtier B et le système d'électrodes 5 peuvent être superposés partiellement, mieux totalement, l'un à l'autre.

Le système d'électrodes 5 comporte de préférence des électrodes du type EcoG utilisant la technologie de l'électrocorticographie décrite précédemment.

Les électrodes du système d'électrodes 5 peuvent être agencées selon une matrice prédéterminée, comme on peut le voir sur la figure 7, par exemple en un réseau de lignes et de colonnes, régulier ou non. En particulier, comme représenté sur la figure 7, le réseau d'électrodes 5 peut être agencé dans une matrice comportant 64 électrodes.

Le système d'électrodes 5 est avantageusement solidaire du boîtier B, étant notamment fixé sur la face inférieure 3l du boîtier B. De la sorte, l'invention peut permettre une maîtrise du positionnement des électrodes sur le cerveau, contrairement aux électrodes selon l'art antérieur réalisées par exemple sur un substrat souple et disposées à distance du boîtier. L'invention peut également permettre d'éviter l'utilisation d'une liaison filaire entre la matrice d'électrodes et le boîtier.

Le système d'électrodes 5 peut être inscrit dans le périmètre externe du boîtier B, notamment dans le périmètre externe de la partie inférieure 3 du boîtier B. De cette façon, il peut y avoir coïncidence entre la zone d'enregistrement des électrodes et la zone accessible du cerveau pour l'enregistrement de signaux neuronaux, formée par la cavité Ca lors d'une craniotomie par exemple.

Selon une variante de réalisation, le système d'électrodes 5 peut comporter des électrodes intracorticales, c'est-à-dire des électrodes aptes à pénétrer dans le cortex, notamment des électrodes reposant sur la technologie LFP décrite précédemment. L'utilisation d'électrodes intracorticales, bien que reposant sur une technologie plus invasive que la technologie de l'électrocorticographie, peut permettre d'obtenir une meilleure résolution spatiale.

En variante encore, le système d'électrodes 5 peut comporter un réseau formé d'électrodes EcoG et d'électrodes intracorticales.

Le positionnement du système d'électrodes 5 peut par exemple être défini en fonction d'examens préalables réalisés sur l'individu.

Le couplage électrique entre le système d'électrodes 5 et les moyens électroniques 4 logés à l'intérieur du boîtier B peut se faire au niveau de passages hermétiques placés au niveau des ouvertures 11 de la partie inférieure 3 décrites précédemment. Chaque passage hermétique peut comporter un corps métallique, notamment un corps de titane, par exemple une lame, muni d'une ou plusieurs ouvertures. Chaque ouverture peut comporter une broche métallique, notamment réalisée en platine ou platine iridiée, laquelle peut être gainée par du verre ou une céramique, par exemple du rubis de façon à permettre d'assurer l'isolation électrique entre la broche métallique et le corps métallique. Dans l'exemple de réalisation représenté, chaque passage hermétique comporte 23 ouvertures, et donc 23 broches. Au niveau des interfaces entre le corps métallique et le gainage, par exemple entre le titane et le rubis, et entre la broche et le gainage, par exemple entre le platine et le rubis, de l'or peut être déposé de façon à permettre d'assurer l'herméticité. Les passages peuvent en outre être noyés dans un matériau de surmoulage, par exemple une résine époxy ou acrylique isolante.

Selon un autre aspect de l'invention, le dispositif implantable 1 comporte une enveloppe 6 de transmission de signaux. Cette enveloppe 6 a pour fonction la transmission de signaux de et/ou vers les moyens électroniques 4 par une liaison sans fil.

L'enveloppe 6 peut s'étendre depuis la périphérie du dispositif 1, et notamment depuis la périphérie de la partie inférieure 3 du boîtier B, comme représenté sur la figure 1.

A la différence du boîtier B, l'enveloppe 6 peut être réalisée dans un matériau souple et biocompatible. L'enveloppe 6 peut par exemple être réalisée à partir d'une résine silicone, par exemple la résine silicone commercialisée par la société NUSIL sous la référence MED6210 ou MED6215.

L'enveloppe 6 est destinée à être insérée entre le crâne C et la peau P du cuir chevelu, dans le prolongement de la surface supérieure 2a du boîtier B, comme on peut le voir sur les figures 9 et 10.

L'enveloppe 6 peut présenter une épaisseur e suffisamment mince pour pouvoir être glissée entre le crâne C et la peau P du cuir chevelu. L'épaisseur e de l'enveloppe 6 peut notamment diminuer en éloignement du boîtier B. Autrement dit, l'épaisseur e de l'enveloppe 6 peut diminuer avec la distance par rapport à la périphérie du boîtier B.

L'épaisseur e de l'enveloppe 6 peut par exemple être comprise entre 1 et 3 mm, étant par exemple inférieure à 1,5 mm, mieux 1 mm, mieux encore 0,7 mm. L'épaisseur e de l'enveloppe 6 peut par exemple être constante sur une distance prédéterminée à partir du boîtier B, puis diminuer progressivement jusqu'à l'extrémité de l'enveloppe 6. Dans l'exemple considéré, l'enveloppe 6 présente par exemple une épaisseur e d'environ 1,5 mm sur une distance A maximale de 3 cm par rapport au boîtier B, puis l'épaisseur e diminue progressivement pour atteindre 0.2 mm à l'extrémité de l'enveloppe 6.

L'enveloppe 6 peut présenter une dureté Shore A égale à environ 50. L'enveloppe 6 peut ainsi avoir une souplesse permettant une bonne adaptation lors de l'implantation entre le crâne C et le cuir chevelu.

L'enveloppe 6 peut présenter une surface supérieure 6a sensiblement convexe (ou courbe) de façon à reproduire au moins partiellement la courbure du crâne et celle du cuir chevelu à l'endroit de son implantation, et à assurer une sensible continuité de surface entre la face supérieure 2a du boîtier B et la surface supérieure 6a de l'enveloppe 6. En particulier, la surface supérieure 6a de l'enveloppe 6 peut être reliée à la face supérieure 2a du boîtier B de façon continue de sorte à présenter un arrondi épousant la forme du cuir chevelu et celle du crâne à l'interface entre le boîtier B et l'enveloppe 6. La surface supérieure 6a de l'enveloppe 6 et la face supérieure 2a du boîtier B peuvent être tangentes l'une par rapport à l'autre au niveau de leur interface. L'invention peut ainsi permettre de minimiser les risques de liaisons et/ou d'infection, notamment de la peau, au niveau de l'interface entre l'enveloppe 6 et le boîtier B.

L'enveloppe 6 peut comporter au moins une antenne de transmission, pour l'échange d'informations en émission et/ou réception, et/ou au moins une antenne de téléalimentation. En particulier, l'enveloppe 6 peut comporter au moins une antenne de transmission de liaison hertzienne pour communiquer avec l'extérieur. Lorsque l'enveloppe 6 comporte deux antennes, notamment une antenne de transmission et une antenne de téléalimentation, les deux antennes sont de préférence coplanaires et disposées de sorte que l'une est inscrite dans l'autre. Le fait d'avoir des antennes coplanaires et inscrites l'une dans l'autre peut permettre de limiter significativement l'épaisseur totale de l'enveloppe 6.

La ou les antennes peuvent être raccordées aux moyens électroniques 4 par l'intermédiaire d'une ouverture 11 de la partie inférieure 3 telle que décrite précédemment ou par un passage rendu étanche par une résine isolante biocompatible.

Dans l'exemple de réalisation décrit, l'enveloppe 6 comporte avantageusement une antenne de téléalimentation 7 et une antenne de transmission 8 des signaux neuronaux traités par les moyens électroniques 4, les deux antennes étant coplanaires et l'antenne de téléalimentation 7 encadrant l'antenne de transmission 8, celle-ci étant inscrite dans l'antenne de téléalimentation 7.

Plus particulièrement, l'enveloppe 6 comporte une antenne de téléalimentation 7 Haute Fréquence (HF) de 13,56 MHz et une antenne de transmission 8 Ultra Haute Fréquence (UHF) de 402 à 405 MHz.

Grâce à l'invention, l'éloignement de la ou des antennes contenues dans l'enveloppe 6 par rapport au boîtier B logeant les moyens électroniques 4 peut permettre de limiter les effets d'écrantage du matériau constituant le boîtier B, c'est-à-dire l'effet d'atténuation du champ électrique en raison de la présence de porteurs de charges électriques mobiles au sein du matériau du boîtier B. En effet, l'enveloppe 6 étant réalisée en un matériau non métallique, par exemple un surmoulage de silicone dans lequel la ou les antennes sont noyées, et le boîtier B étant réalisé en un matériau métallique, notamment en titane, l'éloignement de l'enveloppe 6 par rapport au boîtier B permet de limiter un effet d'écrantage pouvant se produire. En outre, le fait de placer la ou les antennes sous le cuir chevelu peut permettre la transmission d'informations provenant de l'extérieur et/ou en direction de l'extérieur tout en minimisant l'atténuation.

Comme on peut le voir sur la figure 1, la distance maximale l₁ entre le boîtier B et l'extrémité de l'antenne de téléalimentation 7 peut être d'environ 3 cm. De même, la distance maximale l₂ entre le boîtier B et l'extrémité de l'antenne de transmission 8 peut être d'environ 1,5 cm.

La largeur (ou écartement) maximale L₁ de l'antenne de téléalimentation 7 peut être d'environ 4 cm. De même, la largeur maximale L₂ de l'antenne de transmission 8 peut être d'environ 3 cm.

Chaque antenne peut comporter un fil métallique, notamment un fil de platine, d'un diamètre par exemple égal à 500 µm. Chaque antenne peut délimiter une surface donnée. Par exemple, l'antenne de téléalimentation 7 peut s'étendre sur une surface d'environ 10 cm².

Les figures 8a et 8b illustrent respectivement les deux étapes de fabrication de l'enveloppe 6 du dispositif 1 selon l'invention.

Au cours de l'étape illustrée sur la figure 8a, chaque antenne 7 et 8 est recouverte d'un premier surmoulage 9', par exemple à l'aide d'une résine silicone, de façon à rigidifier l'antenne. Le surmoulage 9' peut consister en une gaine d'un diamètre d'environ de 1.5 mm autour du fil constituant l'antenne.

Au cours de l'étape illustrée sur la figure 8b, chaque antenne est recouverte d'un deuxième surmoulage 9, par exemple avec le même matériau que pour le premier surmoulage 9', afin d'obtenir l'enveloppe 6. Le deuxième surmoulage 9 peut être effectué sur l'intégralité des antennes 7 et 8 et de l'espace séparant les antennes 7 et 8.

Bien entendu, l'invention n'est pas limitée à l'exemple de réalisation qui vient d'être décrit.

En particulier, le système d'électrodes 5 peut comporter des électrodes de stimulation, les signaux électriques de stimulation étant alors générés par les moyens électroniques 4 situés dans le boîtier B. Ces moyens peuvent alors comporter des générateurs de signaux de stimulation.

L'expression « comportant un » doit être comprise comme étant synonyme de « comportant au moins un », sauf si le contraire est spécifié.

## Revendications

1. Dispositif (1) implantable du type interface neuronale pour le traitement de signaux, comportant :
- un boîtier (B) destiné à être logé au moins partiellement dans une cavité (Ca) formée sur le crâne (C) d'un être humain ou animal, le boîtier (B) comportant une face supérieure (2a), destinée à venir au contact de la peau (P), dont la surface est convexe,
- des moyens électroniques (4) de traitement de signaux situés dans le boîtier (B), et
- un système d'électrodes (5) couplé électriquement aux moyens électroniques (4),
**caractérisé en ce que** le dispositif (1) comporte une enveloppe (6) de transmission de signaux, s'étendant depuis la périphérie du boîtier (B), l'enveloppe (6) présentant une surface supérieure (6a) convexe de façon à reproduire au moins partiellement la courbure du crâne (C) et/ou celle du cuir chevelu à l'endroit de son implantation, et à assurer une sensible continuité de surface entre la face supérieure (2a) du boîtier (B) et la surface supérieure (6a) de l'enveloppe (6),
**en ce que** la surface supérieure (6a) de l'enveloppe (6) et la face supérieure (2a) du boîtier (B) sont tangentes l'une par rapport à l'autre au niveau de leur interface,
et **en ce que** la surface supérieure (6a) de l'enveloppe (6) est reliée à la face supérieure (2a) du boîtier (B) de façon continue de sorte à présenter un arrondi épousant la forme du cuir chevelu et/ou celle du crâne (C) à l'interface entre le boîtier (B) et l'enveloppe (6).

2. Dispositif selon la revendication 1, **caractérisé en ce que** l'enveloppe (6) comporte au moins une antenne de transmission (8) et/ou une antenne de téléalimentation (7).

3. Dispositif selon l'une quelconque des revendications précédentes, **caractérisé en ce que** le système d'électrodes (5) est inscrit dans le périmètre externe du boîtier (B).

4. Dispositif selon l'une quelconque des revendications précédentes, **caractérisé en ce que** l'enveloppe (6) comporte une antenne de transmission (8) et une antenne de téléalimentation (7), les deux antennes étant coplanaires et disposées de sorte que l'une (8) est inscrite dans l'autre (7).

5. Dispositif selon l'une quelconque des revendications précédentes, **caractérisé en ce que** l'épaisseur (e) de l'enveloppe (6) diminue en éloignement du boîtier (B).

6. Dispositif selon l'une quelconque des revendications précédentes, **caractérisé en ce que** l'épaisseur (e) de l'enveloppe (6) est comprise entre 1 mm et 3 mm.

7. Dispositif selon l'une quelconque des revendications précédentes, **caractérisé en ce que** l'enveloppe (6) présente une dureté Shore A inférieure ou égale à 50.

8. Dispositif (1) selon l'une quelconque des revendications précédentes, **caractérisé en ce que** le boîtier (B) est formé par l'assemblage d'une partie supérieure (2) et d'une partie inférieure (3).

9. Dispositif (1) selon l'une quelconque des revendications précédentes, **caractérisé en ce que** le système d'électrodes (5) comporte des électrodes d'enregistrement de signaux neuronaux et/ou des électrodes de stimulation, et **en ce que** les moyens électroniques (4) comportent des moyens de traitement de signaux neuronaux mesurés par les électrodes d'enregistrement et/ou des moyens de production de signaux d'électrostimulation par les électrodes de stimulation.

10. Dispositif (1) selon l'une quelconque des revendications précédentes, **caractérisé en ce que** le boîtier (B) est réalisé dans un matériau rigide et biocompatible, et **en ce que** l'enveloppe (6) est réalisée dans un matériau souple et biocompatible.

11. Procédé de fabrication d'un dispositif (1) selon l'une quelconque des revendications 1 à 10, dans lequel on détermine la convexité de la face supérieure (2a) du boîtier (B) du dispositif (1) à partir de mesures de convexité préalablement effectuées sur le crâne (C) d'un être humain ou animal.

## Patentansprüche

1. Implantierbare Vorrichtung (1) vom Typ einer neuronalen Schnittstelle für die Verarbeitung von Signalen, umfassend:
- ein Gehäuse (B), welches vorgesehen ist, wenigstens teilweise in einer Kavität (Ca) aufgenommen zu sein, welche am Schädel (C) eines menschlichen oder tierischen Wesens gebildet ist, wobei das Gehäuse (B) eine obere Seite (2a) umfasst, welche vorgesehen ist, in Kontakt mit der Haut (P) zu kommen, deren Oberfläche konvex ist,
- elektronische Mittel (4) zur Verarbeitung von Signalen, welche in dem Gehäuse (B) angeordnet sind, und
- ein System von Elektroden (5), welches elektrisch mit den elektronischen Mitteln (4) gekoppelt ist,
**dadurch gekennzeichnet, dass** die Vorrichtung (1) eine Hülle (6) zur Übertragung von Signalen umfasst, welche sich von der Peripherie des Gehäuses (B) erstreckt, wobei die Hülle (6) eine derartige konvexe obere Oberfläche (6a) aufweist, dass wenigstens teilweise die Krümmung des Schädels (C) und/oder diejenige der Kopfhaut an der Position ihrer Implantation nachgebildet wird und eine fühlbare Stetigkeit der Oberfläche zwischen der oberen Seite (2a) des Gehäuses (B) und der oberen Oberfläche (6a) der Hülle (6) sichergestellt wird,
dass die obere Oberfläche (6a) der Hülle (6) und die obere Seite (2a) des Gehäuses (B) auf dem Niveau ihres Verbindungsbereichs Tangenten zueinander sind,
und dass die obere Oberfläche (6a) der Hülle (6) an der oberen Seite (2a) des Gehäuses (B) in durchgehender Weise angebracht ist, um eine Rundung aufzuweisen, welche sich der Form der Kopfhaut und/oder derjenigen des Schädels (C) an dem Verbindungsbereich zwischen dem Gehäuse (B) und der Hülle (6) anpasst.

2. Vorrichtung nach Anspruch 1, **dadurch gekennzeichnet, dass** die Hülle (6) wenigstens eine Sendeantenne (8) und/oder eine Fernspeise-Antenne (7) umfasst.

3. Vorrichtung nach einem der vorhergehenden Ansprüche, **dadurch gekennzeichnet, dass** das System von Elektroden (5) in dem Außenumfang des Gehäuses (B) aufgenommen ist.

4. Vorrichtung nach einem der vorhergehenden Ansprüche, **dadurch gekennzeichnet, dass** die Hülle (6) eine Sendeantenne (8) und eine Fernspeise-Antenne (7) umfasst, wobei die beiden Antennen koplanar und derart angeordnet sind, dass die eine (8) in der anderen (7) aufgenommen ist.

5. Vorrichtung nach einem der vorhergehenden Ansprüche, **dadurch gekennzeichnet, dass** die Dicke (e) der Hülle (6) sich von dem Gehäuse (B) entfernend abnimmt.

6. Vorrichtung nach einem der vorhergehenden Ansprüche, **dadurch gekennzeichnet, dass** die Dicke (e) der Hülle (6) zwischen 1 mm und 3 mm beträgt.

7. Vorrichtung nach einem der vorhergehenden Ansprüche, **dadurch gekennzeichnet, dass** die Hülle eine Shore-A-Härte von kleiner oder gleich 50 aufweist.

8. Vorrichtung (1) nach einem der vorhergehenden Ansprüche, **dadurch gekennzeichnet, dass** das Gehäuse (B) durch Verbinden eines oberen Teils (2) und eines unteren Teils (3) gebildet ist.

9. Vorrichtung (1) nach einem der vorhergehenden Ansprüche, **dadurch gekennzeichnet, dass** das System von Elektroden (5) Elektroden zum Aufzeichnen von neuronalen Signalen und/oder Elektroden zur Stimulation umfasst, und dass die elektronischen Mittel (4) Mittel zum Verarbeiten von neuronalen Signalen, welche durch die Elektroden zum Aufzeichnen gemessen worden sind, und/oder Mittel zum Erzeugen von Signalen zur Elektrostimulation durch die Elektroden zur Stimulation, umfassen.

10. Vorrichtung (1) nach einem der vorhergehenden Ansprüche, **dadurch gekennzeichnet, dass** das Gehäuse (B) aus einem steifen und biokompatiblen Material hergestellt ist, und dass die Hülle (6) aus einem weichen und biokompatiblen Material hergestellt ist.

11. Verfahren zum Herstellen einer Vorrichtung (1) nach einem der Ansprüche 1 bis 10, wobei die Konvexität der oberen Seite (2a) des Gehäuses (B) der Vorrichtung (1) ausgehend von Messungen der Konvexität bestimmt wird, welche zuvor an dem Schädel (C) eines menschlichen oder tierischen Wesens durchgeführt worden sind.

## Claims

1. An implantable device (1) of the neural interface type for the processing of signals, including:
- a case (B) intended to be held at least partially in a cavity (Ca) formed on the cranium (C) of a human or animal, said case (B) including an upper face (2a), intended to come into contact with the skin (P), the surface of which is convex,
- an electronic circuit (4) for processing signals positioned in the case (B), and
- a system of electrodes (5) coupled electrically to the electronic circuit (4),
**characterized in that** the device (1) includes a signal transmission sleeve (6), extending from the periphery of the case (B), the sleeve (6) having a convex upper surface (6a) such that it at least partially reproduces the curvature of the cranium (C) and/or that of the scalp in the area where it is implanted, and such that it provides an appreciable surface continuity between the upper face (2a) of the case (B) and the upper face (6a) of the sleeve (6),
**in that** the upper face (6a) of the sleeve (6) and the upper face (2a) of the case (B) are tangential relative to one another at their interface, and **in that** the upper surface (6a) of the sleeve (6) is connected to the upper face (2a) of the case (B) in continuous fashion, such that it has a roundness closely following the shape of the scalp and/or that of the cranium (C) at the interface between the case (B) and the sleeve (6).

2. The device according to claim 1, wherein the sleeve (6) includes at least one transmission antenna (8) and/or one remote power feeding antenna (7).

3. The device according to any preceding claim , wherein the system of electrodes (5) is contained in the external perimeter of the case (B).

4. The device according to any preceding claim, wherein the sleeve (6) includes a transmission antenna (8) and a remote power feeding antenna (7), where both antennae are coplanar and positioned such that one (8) is contained within the other (7).

5. The device according to any preceding claim, wherein the thickness (e) of the sleeve (6) is less the further it is from the case (B).

6. The device according to any preceding claim, wherein the thickness (e) of the sleeve (6) is between 1 mm and 3 mm.

7. The device according to any preceding claim, wherein the sleeve (6) has a Shore A hardness less or equal to 50.

8. The device according to any preceding claim, wherein the case (B) is formed by assembling an upper portion (2) and a lower portion (3).

9. The device according to any preceding claim, wherein the system of electrodes (5) includes electrodes for recording neural signals and/or stimulation electrodes, and wherein the electronic circuit (4) include means for processing neural signals measured by the recording electrodes and/or means of production of electrical stimulation signals by the stimulation electrodes.

10. The device according to any preceding claim, wherein the case (B) is made from a rigid and biocompatible material, and wherein the sleeve (6) is made from a flexible and biocompatible material.

11. A method for manufacturing a device (1) according to any one of claims 1 to 10, wherein the convexity of the upper face (2a) of the case (B) of the device (1) is determined from convexity measurements previously made on the cranium (C) of a human or animal.
